(19) Europäisches Patentamt
European Patent Office
Office européen des brevets

(11) Publication number: **0 187 332**
**B1**

(12) **EUROPEAN PATENT SPECIFICATION**

(45) Date of publication of patent specification: **01.02.89**

(51) Int. Cl.$^4$: **C 12 Q 1/68** // G01N33/532

(21) Application number: **85116199.2**

(22) Date of filing: **18.12.85**

# (54) Photochemical method of labelling nucleic acids for detection in hybridization assays.

(30) Priority: **10.01.85 US 690336**

(43) Date of publication of application:
**16.07.86 Bulletin 86/29**

(45) Publication of the grant of the patent:
**01.02.89 Bulletin 89/05**

(84) Designated Contracting States:
**AT BE CH DE FR GB IT LI LU NL SE**

(56) References cited:
**EP-A-0 124 221**
**EP-A-0 130 515**
**EP-A-0 131 830**
**EP-A-0 138 357**
**EP-A-0 156 287**
**WO-A-85/02628**

(73) Proprietor: **Molecular Diagnostics, Inc.**
**400 Morgan Lane**
**West Haven, CT.06516 (US)**

(72) Inventor: **Dattagupta, Nanibhushan**
**470 Prospect Street**
**New Haven, CT 06511 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This application relates to the provision of labeled nucleic acid labeled probes suitable for hybridization assays.

In European Patent Application No. 84107624.3 there is described a photochemical method of preparing labeled nucleic acids. The assays are of the type described in the European Application No. 84107248.1.

The labeled probes of EP 84107624.3 comprise (a) a nucleic acid component, (b) a nucleic acid-binding ligand photochemically linked to the nucleic acid component, and (c) a label chemically linked to (b). Further, in EP—A—156287 and in WO—A—85/02628, both applications filed earlier than, but published after priority of the present application, there are described labeled nucleic acid probes wherein the spacer molecule includes a chain of up to 40 atoms composed of carbon, oxygen, nitrogen and sulfur. Those probes generally perform quite satisfactorily but in some instances it is desirable to improve their performance, for example, it is an object of the present invention to provide a probe of improved solubility, to increase the efficiency of preparing such probes, and to improve the sensitivity of the probe in assay.

These and other objects and advantages are realized in accordance with the present invention pursuant to which there is provided a labeled nucleic acid probe comprising (a) a nucleic acid component (b) a nucleic acid-binding ligand photochemically linked to the nucleic acid component, (c) a label and (d) a spacer chemically linking (b) and (c).

Such spacer is a member selected from the group consisting of -glycyl-glycyl-glycyl-, an -amino-alkane-carbonyl radical such as $—NH(CH_2)_5—CO—$, a spermine or spermidine radical, an w-alkanediamine radical such as $—NH—(CH_2)_6—NH$ or $—HN—CH_2—CH_2—NH$. These spacers can be directly linked to the nucleic acid-binding ligand and/or the label or the linkages may include a divalent radical of a coupler such as dithiobis succinimidyl propionate, 1,4-butanediol diglycidyl ether, a diisocyanate, carbodiimide, glyoxal, glutaraldehyde or the like.

The spacer can be incorporated at any stage of the process of making the probe

a–b–d–c

defined hereinabove. Thus the sequence can be any of the following:

a+b+d+c,

b+d+c+a,

d+c+b+a,

b+d+a+c, etc.

The conditions for the individual steps are well known in chemistry.

As described in EP 84107624.3, the nucleic acid is joined to the ligand photochemically, employing a photoreactive nucleic as a furocoumarin or a phenanthridine compound or a non-intercalator compound such as netropsin, distamycin, Hoechst 33258 and bis-benzimidazole to link the nucleic acid to a label which can be "read" or assayed in conventional manner, including fluorescence detection. The end product is thus a labeled nucleic acid probe comprising (a) a nucleic acid component, (b) an intercalator or other nucleic acid-binding ligand photochemically linked to the nucleic acid component, and (c) a label chemically linked to (b).

Angelicin derivatives have the following formula

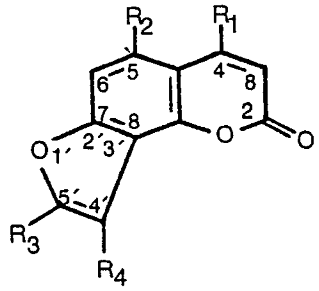

| Parent compound | $R_1$ | $R_2$ | $R_3$ | $R_4$ |
|---|---|---|---|---|
| | H | H | H | H |
| | $CH_3$ | H | $CH_3$ | H |
| | $CH_3$ | $CH_3$ | $CH_3$ | $CH_2OH$ |
| | $CH_3$ | H | $CH_3$ | $CH_2OH_3$ |
| | $CH_3$ | H | $CH_3$ | $CH_2NH_2$ |
| | $CH_3$ | H | $CH_3$ | $CH_2Cl$ |
| | $CH_3$ | H | $CH_3$ | 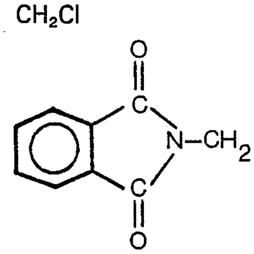 |

Many other compounds with different R's can be synthesised following published procedures.

*Example* with $R_1$ and $R_3$ methyl $R_2$ = H and $R_4$ = $—CH_2NH_2·(RNH_2)$.

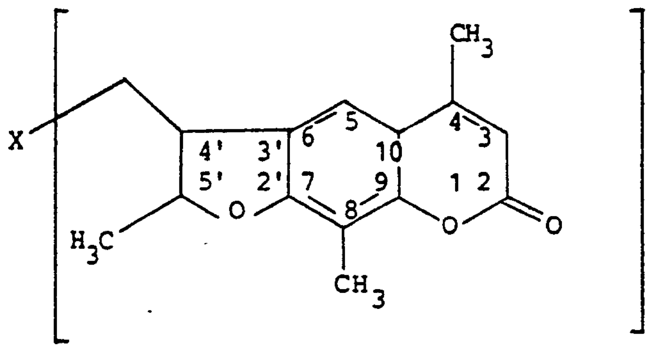

≡ RX

with X = $NH_2$ has been synthesised

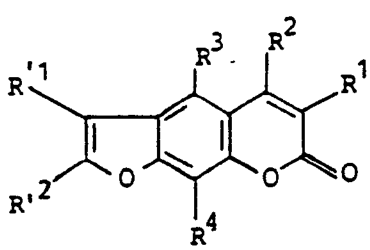

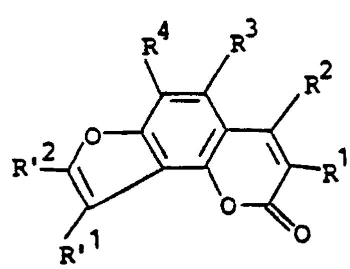

$R_1$—$R_4$ can be —H, —OH, alkyl, alkoxyl, carbonyl, carbalcoxyl
$R'_1$ and $R'_2$ can be —H, —OH, alkyl, alkoxyl, carboxyl, carbalkoxyl and X—Y
where
X is
$—C—(CH_2)_n—Y$
$—N—(CH_2)_n—Y$
$—C—N—(CH_2)_n—Y$
—C—N-polyamine-Y  degree of polyamine = 1—30
—C—N-polyether-Y
—C—N-polyalcohol-Y
—C—S—
—C—O—
Y is a label as biotin.

Reaction of *3* with $R^*NH_2$ where $R^*NH_2$ is

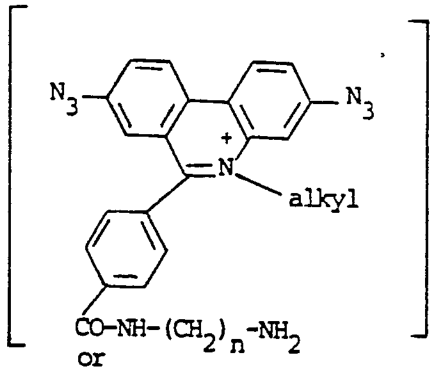

halide

or

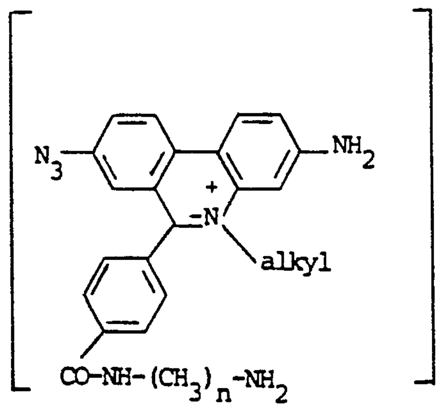

halide

or

Angelicin *s* as in P 11
Amt as in P 12
Daunomycin as in P 13
Amino acridines as in P (14)
Any other intercalator described can be modified similarly to carry a linker and a label for nucleic acids.

*Anthracyclines:* photochemical reactions of anthracyclines are known.

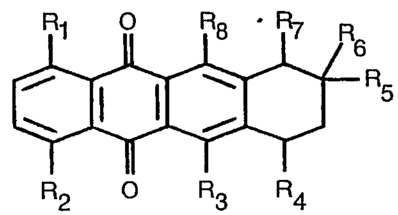

| Compound Name | $R_1$ | $R_2$ | $R_3$ | $R_4$ | $R_5$ | $R_6$ | $R_7$ | $R_8$ |
|---|---|---|---|---|---|---|---|---|
| Daunomycin | H | $OCH_3$ | OH | daunosamine | OH | $COCH_3$ | H | OH |
| Adriamycin | H | $OCH_3$ | OH | daunosamine | OH | $COCH_2OH$ | H | OH |
| Iremycin | H | OH | OH | H | OH | $C_2H_5$ | sugar $N(CH_3)_2$ | OH |
| Violamycin | H | OH | OH | sugar $N(CH_3)_2$ | OH | $C_2H_5$ | sugar $N(CH_3)_2$ | OH |
| Aclacinomycin | H | OH | OH | trisaccharide | OH | $C_2H_5$ | $COOCH_3$ | H |
| Rubidazone | H | $OCH_3$ | OH | daunosamine | OH | 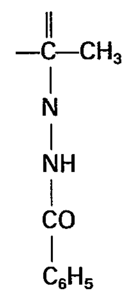 | H | OH |

Many other analogues have been synthesised and found naturally.

The novel photochemical method provides more favorable reaction conditions than the usual chemical coupling method for biochemically sensitive substances. By using proper wavelengths for irradiation, DNA, RNA and proteins can be modified without affecting the native structure of the polymers. The nucleic acid-binding ligand, hereinafter exemplified by an intercalator, and label can first be coupled and then protoreacted with the nucleic acid or the nucleic acid can first be photoreaction with the intercalator and then coupled to the label. A general scheme for coupling a nucleic acid, exemplified by double-stranded DNA, to a label such as a hapten or enzyme is as follows:

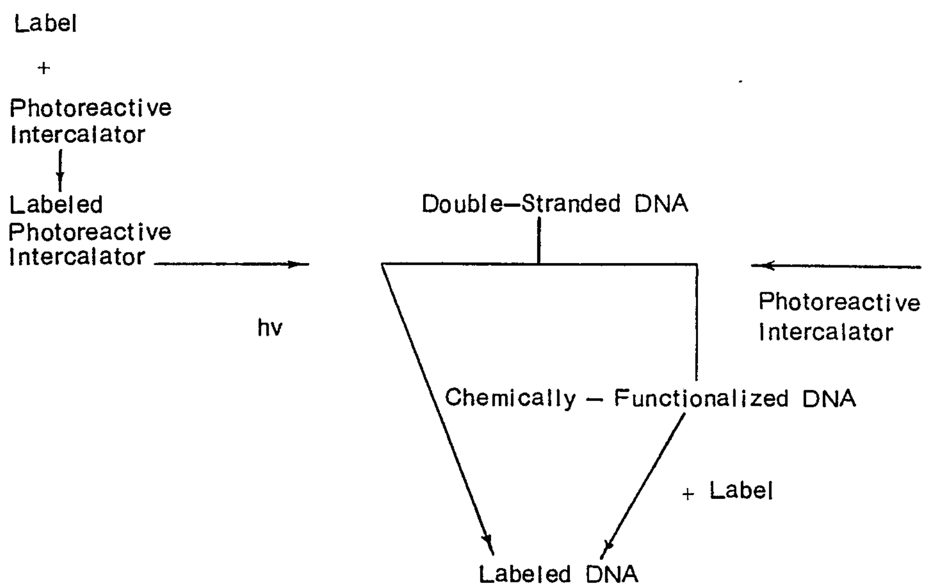


Where the hybridizable portion of the probe is in a double stranded form, such portion is then denaturated to yield a hybridizable single stranded portion. Alternatively, where the labeled DNA comprises the hybridizable portion already in single stranded form, such denaturization can be avoided if desired. Alternatively, double stranded DNA can be labeled by the approach of the present invention after hybridization has occurred using a hybridization format which generates double stranded DNA only in the presence of the sequence to be detected.

To produce specific and efficient photochemical products, it is desirable that the nucleic acid component and the photoreactive intercalator compound be allowed to react in the dark in a specific manner.

For coupling to DNA, aminomethyl psoralen, aminomethyl angelicin and amino alkyl ethidium or methidium azides are particularly useful compounds. They bind to double-stranded DNA and only the complex produces photoadduct. In the case where labeled double-stranded DNA must be denaturated in order to yield a hybridizable single stranded region, conditions are employed so that simultaneous interaction of two strands of DNA with a single photoadduct is prevented. It is necessary that the frequency of modification along a hybridizable single stranded portion of the probe not be so great as to substantially prevent hybridization, and accordingly there preferably will be not more than one site of modification per 25, more usually 50, and preferably 100, nucleotide bases. Angelicin derivatives are superior to psoralen compounds for monoadduct formation. If a single-stranded probe is covalently attached to some extra double-stranded DNA, use of phenanthridum and psoralen compounds is desirable since these compounds interact specifically to double-stranded DNA in the dark. The chemistry for the synthesis of the coupled reagents to modify nucleic acids for labelling, described more fully hereinbelow, is similar for all cases.

The nucleic acid component can be singly or doubly stranded DNA or RNA or fragments there of such as are produced by restriction enzymes or even relatively short oligomers.

The nucleic acid-binding ligands of the present invention used to link the nucleic acid component to the label can be any suitable photoreactive form of known nucleic acid-binding ligands. Particularly preferred nucleic acid-binding ligands are intercalator compounds such as the furocoumarins, e.g., angelicin (isopsoralen) or psoralen or derivatives thereof which photochemically will react with nucleic acids, e.g., 4'-aminomethyl-4,5'-dimethyl angelicin, 4'-aminomethyl-trioxsalen (4'-aminomethyl-4,5', 8-trimethylpsoralen, 3-carboxy-5- or -8-amino- or -hydroxy-psoralen, as well as mono- or bis-azido aminoalkyl methidium or ethidium compounds. Photoreactive forms of a variety of other intercalating agents can also be used as exemplified in the following table:

| *Intercalator Classes and Representative Compounds* | *Literature References* |
|---|---|
| A. Acridine dyes | Lerman, J. Mol. Biol. 3:18 (1961); Bloomfield et al, "Physical Chemistry of Nucleic Acids", Chapter 7, pp. 429—476, Harper and Rowe, NY (1974) |
| proflavin, acridine orange, quinacrine, acriflavine | Miller et al, Biopolymers 19:2091 (1980) |
| B. Phenanthridines | Bloomfield et al, supra |
| ethidium | Miller et al, supra |
| coralyne | Wilson et al, J. Med. Chem. 19:1261 (1976) |
| ellipticine, ellipticine cation and derivatives | Fety et al, FEBS Letters 17:321 (1971); Kohn et al, Cancer Res. 35:71 (1976); LePecq et al, PNAS (USA) 71:5078 (1974); Pelaprat et al, J. Med. Chem. 23:1330 (1980) |
| C. Phenazines<br>5-methylphenazine cation | Bloomfield et al, supra |
| D. Phenothiazines<br>chlopromazine | ibid |
| E. Quinolines<br>chloroquine<br>quinine | ibid |
| F. Aflatoxin | ibid |
| G. Polycyclic hydrocarbons and their oxirane derivatives | ibid |
| 3,4-benzpyrene<br>benzopyrene diol epoxidie, 1-pyrenyl-oxirane | Yange et al, Biochem. Biophys. Res. Comm. 82:929 (1978) |
| benzanthracene-5,6-oxide | Amea et al, Science 176:47 (1972) |
| H. Actinomycins<br>actinomycin D | Bloomfield et al, supra |
| I. Antracyclinones<br>B-rhodomycin A<br>daunamycin | ibid |
| J. Thiaxanthenones<br>miracil D | ibid |
| K. Anthramycin | ibid |
| L. Mitomycin | Ogawa et al, Nucl. Acids REs., Spec. Publ. 3:79 (1977) Akhtar et al, Can. J. Chem. 53:2891 (1975) |
| M. Platinium Complexes | Lippard, Accts. Chem. Res. 11:211 (1978) |

| *Intercalator Classes and Representative Compounds* | *Literature References* |
|---|---|
| N. Polyintercalators<br>echinomycin | Waring et al, Nature 252:653 (19744); Wakelin, Biochem. J. 157:721 (1976) |
| quinomycin<br>triostin<br>BBM928A<br>tandem | Lee et al, Biochem. J. 173:115 (1978); Huang et al, Biochem. 19:5537 (1980): Viswamitra et al, Nature 289:817 (1981) |
| diacridines | LePecq et al, PNAS (USA) 72:2915 (1975): Carrellakis et al, Biochim. Biophys. Acta 418:277 (1976);; Wakelin et al, Biochem 17:5057 (1978); Wakelin et al, FEBS Lett. 104:261 (1979); Capelle et al, Biochem. 18:3354 (1979); Wright et al, Biochem; 19:5825 (1980); Bernier et al, Biochem. J. 199:479 (1981); King et al, Biochem. 21:4982 (1982) |
| ethidium dimer | Gaugain et al, Biochem. 17:5078 (1978); Kuhlman et al, Nucl. Acids Res. 5:2629 (1978); Marlcovits et al, Anal. Biochem. 94:259 (1979): Dervan et al, JACS 100:1968 (1978); ibid 101:3664 (1979). |
| ellipticine dimers and analogs | Debarre et al, Compt. Rend. Ser. D. 284:81 (1977); Pelaprat et al, J. Med. Chem. 23:1336 (1980) |
| heterodimers | Cain et al, J. Med. Chem. 21:658 (1978); Gaugain et al, Biochem. 17:5078 (1978) |
| trimers | Hansen et al, JCS Chem. Comm. 162 (1983); Atnell et al, JACS 105:2913 (1983) |
| O. Norphillin A | Loun et al, JACS 104: 3213 (1982) |
| P. Fluorenes and fluorenones | Bloomfield et al, supra |
| fluorenodiamines | Witkowski et al, Wiss. Beitr.-Martin-Luther-Univ. Halle Wittenberg, 11 (1981) |
| Q. Furocoumarins | |
| angelicin | Venema et al, MGG, Mol. Gen. Genet. 179;1 (1980) |
| 4,5′-dimethylangelicin | Vedaldi et al, Chem.-Biol. Interact. 36:275 (1981) |
| psoralen | Marciani et al, Z. Naturforsch B 27(2):196 (1972) |
| 8-methoxypsoralen | Belognzov et al, Mutat. Res. 84:11 (1981); Scott et al, Photochem. Photobiol. 34:63 (1981) |
| 5-aminomethyl-8-methoxypsoralen | Hansen et al, Tet. Lett. 22:1847 (1981) |
| 4,5,8-trimethylpsoralen | Ben-Hur et al, Biochem. Biophys. Acta 331:181 (1973) |
| 4′-aminomethyl-4,5,8-trimethylpsoralen | Issacs et al, Biochem. 16:1058 (1977) |
| xanthotoxin | Hradecma et al, Acta Virol. (Engl. Ed.) 26:305 (1982) |
| khellin | Beaumont et al, Biochim. Biophys. Acta 608:1829 (1980) |

| *Intercalator Classes and Representative Compounds* | *Literature References* |
|---|---|
| R. Benzodipyrones | Murx et al, J. Het. Chem. 12:417 (1975); Horter et al, Photochem. Photobiol. 20:407 (1974) |
| S. Monostral Fast Blue | Juarranz et al, Acta Histochem. 70:130 (1982) |

Particularly useful photoreactive forms of such intercalating agents are the azidointercalators. Their reactive nitrenes are readily generated at long wavelength ultraviolet or visible light and the nitrenes of arylazides prefer insertion reactions over their rearrangement products [see White et al, Methods in Enzymol. 46:644 (1977)]. Representative azidointercalators are 3-azidoacridine, 9-azidoacridine, ethidium monoazide, ethidium diazide, ethidium dimer azide [Mitchell et al, JACS 104:4265 (1982)], 4-azido-7-chloroquinoline, and 2-azidofluorene. Other useful photoreactable intercalators are the furocoumarins which form [2+2] cycloadducts with pyrimidine residues. Alkylating agents can also be used such as bis-chloroethylamines and epoxides or aziridines, e.g., aflatoxins, polycyclic hydrocarbon epoxides, mitomycin, and norphillin A.

The label which is linked to the nucleic acid component according to the present invention can be any chemical group or residue having a detectable physical or chemical property. The label will bear a functional chemical group to enable it to be chemically linked to the intercalator compound. Such labeling materials have been well developed in the field of immunoassays and in general most any label useful in such methods can be applied to the present invention. Particularly useful are enzymatically active groups, such as enzymes (see Clin. Chem. (1976) 22:1243), enzyme substrates (see British Pat. Spec. 1,548,741), coenzymes (see U.S. Pat. Nos. 4,230,797 and 4,238,565), and enzyme inhibitors (see U.S. Pat. No. 4,134,792; fluorescers (see Clin. Chem. (1979) 25:353) and chromophores including phycobiliproteins; luminescers such as chemiluminescers and bioluminescers (see Clin. Chem. (1979) 25:512, and ibid, 1531); specifically bindable ligands; and residues comprising radioisotopes such as $^{3}H$, $^{35}S$, $^{32}P$, $^{125}I$, and $^{14}C$. Such labels are detectd on the basis of their own physical properties (e.g., fluorescers, chromophores and radioisotopes) or their reactive or binding properties (e.g., enzymes, substrates, coenzymes and inhibitors). For example, a cofactor-labeled nucleic acid can be detected by adding the enzyme for which the label is a cofactor and a substrate for the enzyme. A hapten or ligand (e.g., biotin) labeled nucleic acid can be detected by adding an antibody or an antibody fragment to the hapten or a protein (e.g., avidin) which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include β-galactosidase, alkaline phosphatase, papain, and peroxidase. For in situ hybridization studies, ideally the final product is water insoluble. Other labels will be evident to one of the ordinary skill in the art.

The label will be linked to the intercalator compound by direct chemical linkage such as involving covalent bonds, or by indirect linkage such as by the incorporation of the label in a microcapsule or liposome which in turn is linked to the intercalator compound. Methods by which the label is linked to the intercalator compound are essentially known in the art and any convenient method can be used to perform the present invention.

Advantageously the intercalator compound is first combined with the label chemically and thereafter combined with the nucleic acid component. For example, since biotin carries a carboxyl group it can be combined with a furocoumarin by way of amide or ester formation without interfering with the photochemical reactivity of the furocoumarin or the biological activity of the biotin, e.g.,

(i) 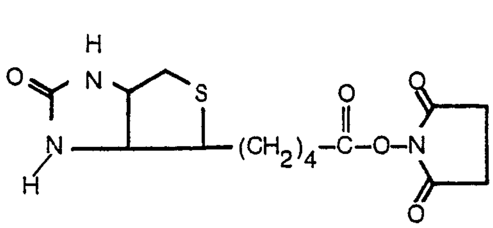

Biotin–N–hydroxysuccinimide

or + $RNH_2$ ⟶

(ii)

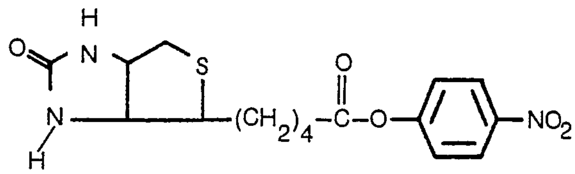

Biotin—p—nitrophenyl ester

(iii)

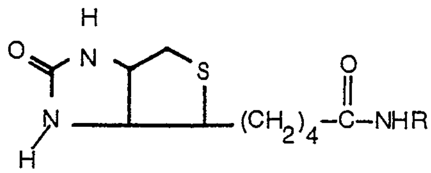

or

Biotin + ROH $\xrightarrow{\text{carbodiimide}}$ Biotin CO OR

By way of example,

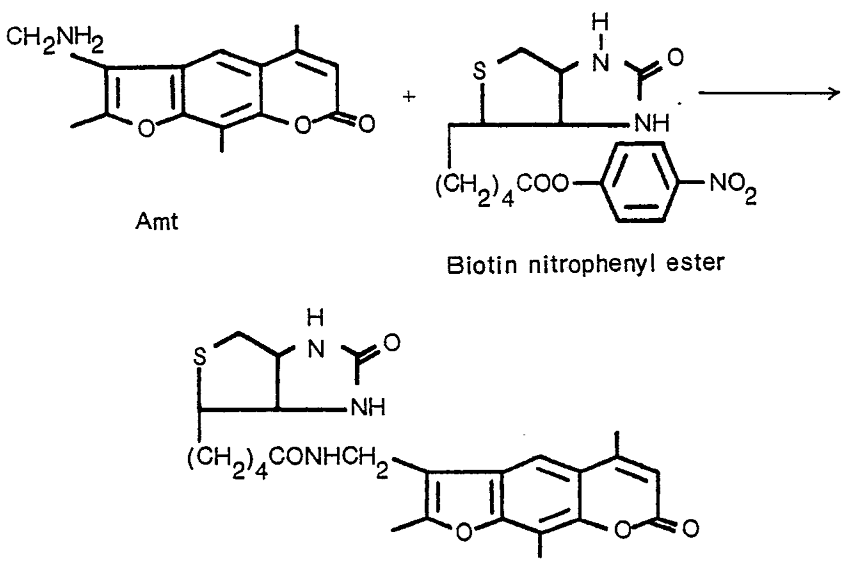

Amt

Biotin nitrophenyl ester

Other aminomethylangelicin, psoralen and phenanthridium derivatives can be similarly reacted, as can phenanthridium halides and derivatives thereof such as aminopropyl methidium chloride, i.e.

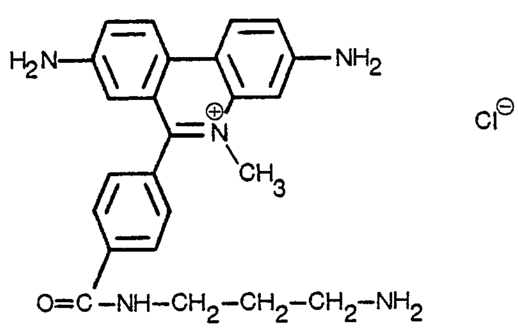

[see Hertzberg et al, J. Amer. Chem. Soc. 104:313 (1982)]

Alternatively a bifunctional reagent such as dithiobis succinimidyl propionate or 1,4-butanediol diglycidyl ether can be used directly to couple the photochemically reactive molecule with the label where the reactants have alkyl amino residues, again in a known manner with regard to solvents, proportions and

reaction conditions. Certain bifunctional reagents, possibly glutaraldyde may not be suitable because, while they couple, they may modify the nucleic acid and thus interfere with the assay. Routine precautions can be taken to prevent such difficulties.

The particular sequence in making the labeled nucleic acid can be varied. Thus, for example, an amino-substituted psoralen can first be photometrically coupled with a nucleic acid, the product having pendant amino groups by which it can be coupled to the label. Alternatively, the psoralen can first be coupled to a label such as an enzyme and then to the nucleic acid.

The spacer chain length between the nucleic acid-binding ligand and the label can be extended via hydrocarbon or peptide. A typical example involves extending an 8-hydroxy psoralen derivative with an alkyl halide, according to the method described by J. L. DeCout and J. Lhomme, Photochemistry Photobiology, *37*, 155—161 (1983). The haloalkylated derivative is then reacted either with thiol or amines to produce the reactive residue, as has been described by W. A. Saffran et al., Proc. Natl. Acad. Sci., U.S.A., *79*, 4594 (1982).

If the label is an enzyme, for example, the product will ultimately be placed on a suitable medium and the extent of catalysis will be determined. Thus, if the enzyme is a phosphatase the medium could contain nitrophenyl phosphate and one would monitor the amount of nitrophenol generated by observing the color. If the enzyme is a β-galactosidase the medium can contain o-nitrophenyl-D-galacto-pyranoside which also will liberate nitrophenol.

The labeled nucleic acid of the present invention is applicable to all conventional hybridization assay formats, and in general to any format that is possible based on formation of a hybridization product or aggregate comprising the labeled nucleic acid. In particular, the unique labeled probe of the present invention can be used in solution and solid-phase hybridization formats, including, in the latter case, formats involving immobilization of either sample or probe nucleic acids and sandwich formats.

The labeled nucleic acid probe will comprise at least one single stranded base sequence substantially complementary to or homologous with the sequence to be detected. However, such base sequence need not be a single continuous polynucleotide segment, but can be comprised of two or more individual segments interrupted by nonhomologous sequences. These nonhomologous sequences can be linear or they can be self-complementary and form hairpin loops. In addition, the homologous region of the probe can be flanked at the 3'- and 5'-terminii by nonhomologous sequences, such as those comprising the DNA or RNA of a vector into which the homologous sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with sample DNA or RNA. Useful probes include linear or circular probes wherein the homologous probe sequence is in essentially only single stranded form (see particularly, Hu and Messing, Gene 17:271 (1982)].

The labeled probe of the present invention can be used in any conventional hybridization technique. As improvements are made and as conceptually new formats are developed, such can be readily applied to the present labeled probe. Conventional hybridization formats which are particularly useful include those wherein the sample nucleic acids or the polynucleotide probe is immobilized on a solid support (solid-phase hybridization) and those wherein the polynucleotide species are all in solution (solution hybridization).

In solid-phase hybridization formats, one of the polynucleotide species participating in hybridization is fixed in an appropriate manner in its single stranded form to a solid support. Useful solid supports are well known in the art and include those which bind nucleic acids either covalently or non-covalently. Noncovalent supports which are generally understood to involve hydrophobic bonding include naturally occurring and synthetic polymeric materials, such as nitrocellulose, derivatized nylon, and fluorinated polyhydrocarbons, in a variety of forms such as filters or solid sheets. Covalent binding supports are also useful and comprise materials having chemically reactive groups or groups, such as dichlorotriazine, diazobenzyloxymethyl, and the like, which can be activated for binding to polynucleotides.

A typical solid-phase hybridization technique begins with immobilization of sample nucleic acids onto the support in single stranded form. This initial step essentially prevents reannealing of complementary strands from the sample and can be used as a means for concentrating sample material on the support for enhanced detectability. The polynucleotide probe is then contacted with the support and hybridization detected by measurement of the label as described herein. The solid support provides a convenient means for separating labeled probe which has hybridized to the sequence to be detected from that which has not hybridized.

Another method of interest is the sandwich hybridization technique wherein one of two mutually exclusive fragments of the homologous sequence of the probe is immobilized and the other is labelled. The presence of the polynucleotide sequence of interest results in dual hybridization to the immobilized and labeled probe segments. See Methods in Enzymology 65:468 (1980) and Gene 21:77—85 (1983) for further details.

Instead of using a bifunctional reagent directly to couple the photochemically reactive residues to the label extra linker residues are incorporated. These have the following advantages:

1. Distance between the detectable label from the DNA surface can be altered to optimise the steric constraints.

2. The proper choice of the linker can improve the solubility property of the conjugate.

3. Proper choice of linker can provide specific sites for secondary reactions. As for example a peptide residue can be digested proteoxytically to release the label into solution.

4. Using cationic linker the overall binding affinity of the labelling compound can be increased. This should help improve the efficiency of photoreaction.

The present invention will now be described with reference to the following examples.

## Example 1

Peptide Spacer

Following the merrified synothesis procedure, R. B. Merrifield, *J. Am. Chem. Soci., 85,* 2149 (1963), a polystyrene Ⓟ coupled to glycylglycylglycine (GGG) is synthesized to form the following structure (hereinafter referred to as P—$CH_2$ GGG:

(GGG)

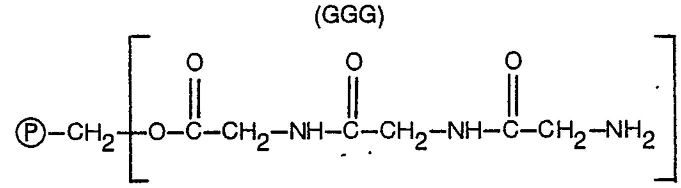

1 g of P-$CH_2$—GGG (approximately 0.5m Moles equivalent of tripeptide (GGG) is suspended in 30 ml of dimethylformamide (DMF), shaken well and to the solution 250 mg solid dicyclohexxylcarbodiimide and 250 mg biotin are added. The resultant mixture is maintained at room temperature (20—25°C) for 15 hrs. The polystyrene resin (P—$CH_2$—GGG linked to biotin) is washed to remove unreacted biotin and other byproducts. The washing is carried out with any solvent which will not attack the crosslinked polystyrene support (P—$CH_2$—GGG) and (for example, washing can be done with DMF, ethanol, etc.)

The product formed is treated with ethanolic sodium hydroxide. The polymer containing the product (GGG—B) formula given below, is treated with a mixture of 20.25 ml ethanol and 2.25 ml aqueous solution of 2 N, sodium hydroxide for 30 minutes at room temperature.

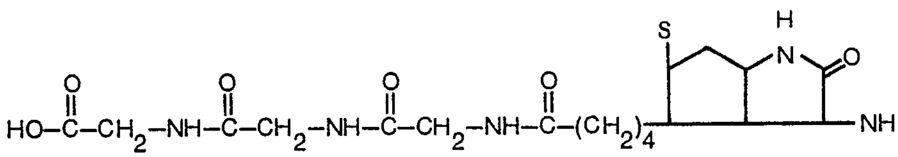

(GGG–B)

The mixture is filtered through fritted disk and immediately neutralized with aqueous sodium hydroxide solution. It is then evaporated to dryness under reduced pressure.

An identical product is also formed by reacting Biotin p-nitrophenyl ester with GGG—B in by DMF. This reaction does not require any carbodicimide.

A reagent containing biotin and an activated peptide linker can be prepared by the following procedure. This reagent can be coupled to any primary aliphatic amine containing molecule. By a slight modification of the above described Merrifield Synthesis, space length 1 to 100 or more atoms can be introduced as spacer molecules between biotin and the nucleic acid binding ligand. The choice of the length of the spacer will be determined by the type of reaction one should carry out to utilize a label e.g., biotin. Instead of biotin reaction with fluorescein isothiocyanate and (FITC) will produce a fluorescein coupled spacer. With FITC the reaction can be carried out after the peptide is released from the support.

## Example 2

Activated Peptide Spacer-Label Compound

100 mg of the compound GGG—B, 50 mg N-hydroxysuccinimide and 30 mg dicyclohexyl carbodiimide are introduced in 10 ml DMF. The solution is shaken at room temperature for 15 hours. The resultant mixture is then cooled to −20°C for 8 hours. This cooling reduces the solubility of the dicyclo-hexylurea. The resultant suspension is centrifugen in a refrigerated centrifuge at 2000 g. The solution is evaporated under reduced pressure to obtain the following compound.

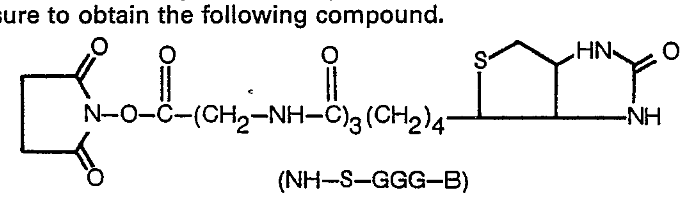

(NH–S–GGG–B)

GGG—B will also react directly with an amine (—$NH_2$) in presence of a diimide.

## Example 3

Hydrocarbon Spacer

The following steps are used to prepare a $(CH_2)_n$ spacer molecule between a label e.g., biotin and a photochemically nucleic acid reactive amine:

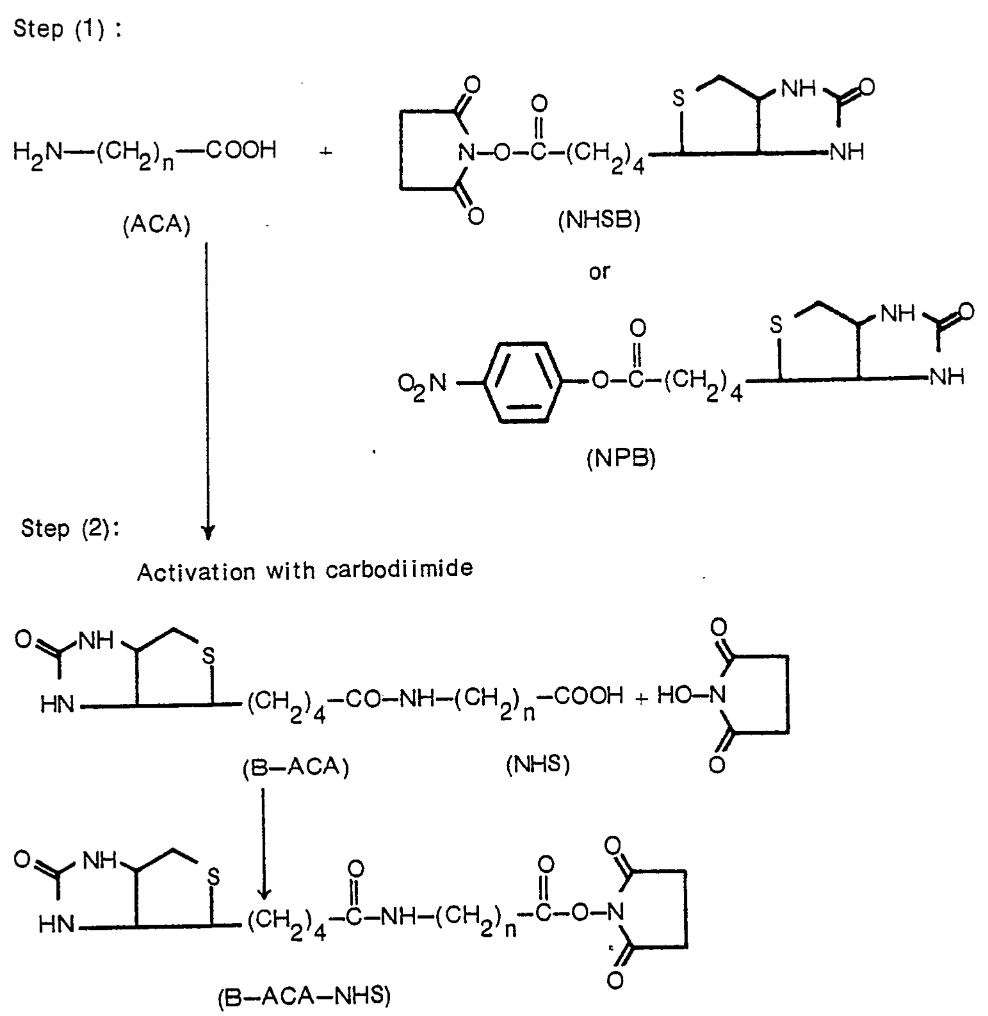


*Preparation of B—ACA (with n = 5)*

0.5 g of 6-aminocaproic acid (n+5, ACA) and 1.5 gm of N-hydroxy-succimidobiotin are introduced in 10 ml dimethyl formamide. The reaction is allowed to proceed for 24 hours at room temperature. DMF is evaporated under reduced pressure. The product B—ACA (n = 5) is ready for reaction with an amine or can be activated with N-hydroxysuccinimide to produce B—ACA—NHS.

*Preparation of B—ACA—NHS (with n = 5)*

80 mg of B—ACA (n = 5), 50 ml N-hydroxysuccinimide and 30 mg dicyclohexylcarbodiimide are mixed in 10 ml DMF. The solution is shaken at room temperature for 15 hours, cooled at −20°C for 4 hours; centrifuger. The solution is evaporated to dryness under reduced pressure to obtain B—ACA—NHS (n = 5). The formed B—ACA—NHS is then ready for reaction with a primary amine.

## Example 4

Polyamine Spacer

The method described below can be used with carboxy psoralen, angelicine, azido phenantridium, azido acidines, and other azido photoreactive compounds with carboxylic (—COOH) residues as the reactive site. A representation of the method is the preparation of MSPB

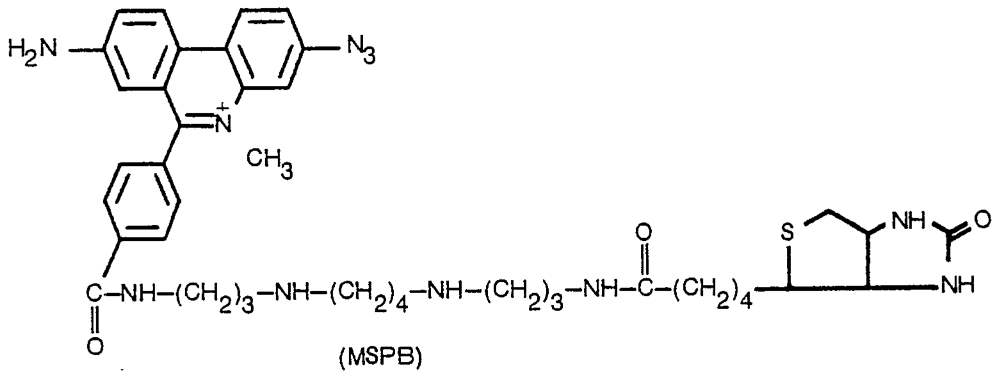

(MSPB)

The acylimidazole ester of monoazido p-carboxymethidium chloride (1 gm) and (5 gms) spermine are introduced in 50 ml dry dimethylsulfoxide as has been described by R. P. Hertzberg and P. Dervan, J. Am. Chem. Soc., 104, 313 (1982) for the reaction with 1:3 diaminopropane. The resulting product formed is reacted with 5 grams of solid N-hydroxysuccinimido biotin.

The MSPB is purified from unreacted materials by column chromatography on a Sephadex LH 20 column using methanol as solvent.

Example 5

Reaction of NHS—GGG—B with 4′-aminomethyl-4,5′ dimethyl angelicin (AMA)

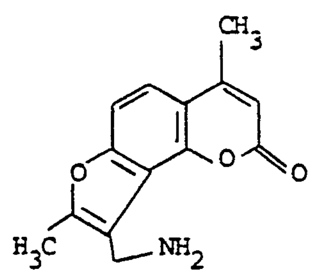

(AMA)

NHS—GGG—B and AMA are mixed in DMF/$H_2O$ (9:1) mixture in a 2:1 molar ratio. The solution is shaken mechanically overnight and then a large excess of glycine is added to deactivate the unreacted ester. The solvent is evaporated under reduced pressure and the compound is chromatographed on a sephadex LH 20 column using methanol-water-(*8:20*) mixture as solvent. The purity of the eluted product (B—GGG—AMA) is determined by TLC (Silica gel chloroform: methanol: acetic acid 8:1:1).

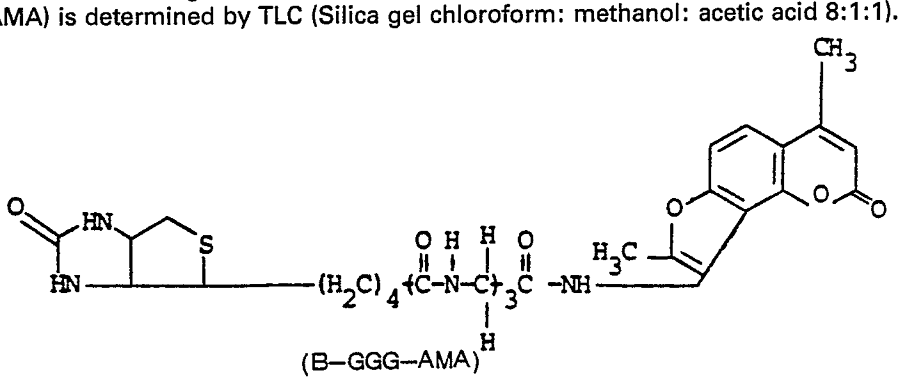

(B–GGG–AMA)

Example 6

Reaction of NHS—GGG—B with 4′-aminomethyl-4,5′,8-trimethylpsoralen (AMT)

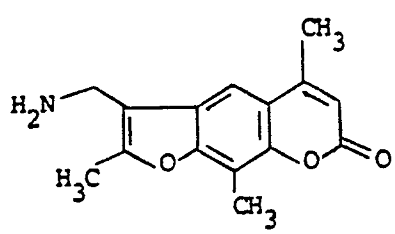

The same procedure as Example 5 is utilized except AMT is used instead of AMA to produce (B—GGG—AMT).

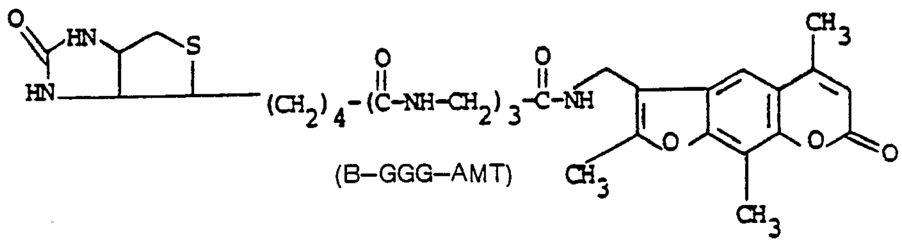

(B–GGG–AMT)

Example 7

Reaction of B—AGA—NHS with AMT or AMA

An identical procedure as Example 5 was utilized, except NHS—AGA—B is used instead of NHS—GGG—B to produce compounds B—AGA—AMT and B—AGA—AMA.

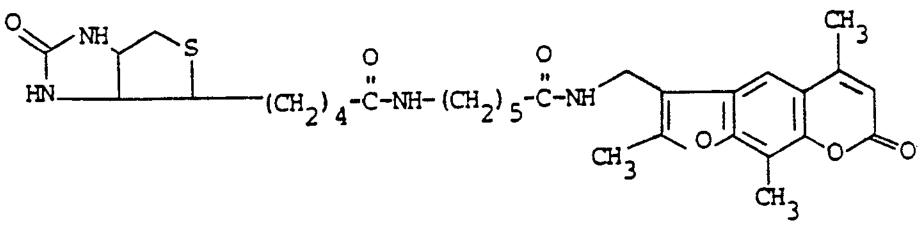

(B–AGA–AMT)

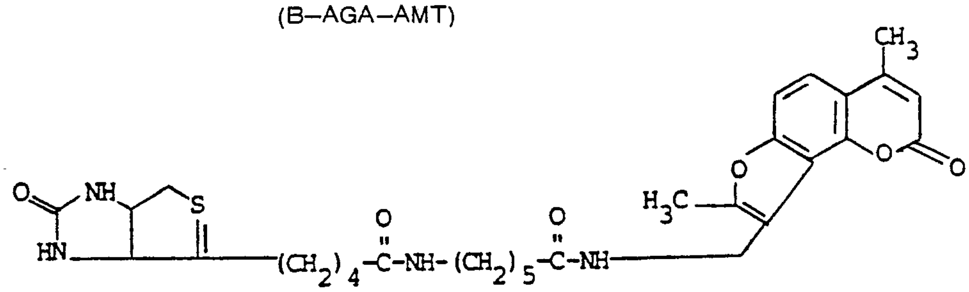

(B–AGA–AMA)

Example 8

Photochemical coupling of B—GGG—AMA to a DNA probe and its use in a hybridization assay:

100 mg of E. Coli DNA is dissolved in 1 ml tris EDTA (10 mm tris-1mM EDTA; CH 7.2) to the DNA solution 10 mg B—GGG—AMA (10 ml solution of 1 mg/ml) is added. The resultant mixture is irradiated at 346 nm for 30 minutes. The mixture is then dialyzed to remove any unreacted B—GGG—AMA. The sample is then precipitated with ethanol. The labeled probe is then tested for its ability to hybridize with E. Coli DNA (test sample).

E. Coli DNA (1 mg) is immobilized onto nitrocellulose paper (refer European Patent Application No. 841 07 248.1) hybridized with the labeled probe.

The test DNA sample is treated with 0.1 M NaOH for 5 minutes, then chilled in ice. The sample is neutralized with an equal volume of 0.1 N HCl, 0.9 NaCl, 0,09 M Na citrate, then filtered under mild aspiration through a nitrocellulose filter (e.g., BA 85 from Schleicher and Schuell) that had been presoaked in 0.9 M NaCl, 0.09 M Na citrate. The filter is then washed with 6 × SSC, (6 × SSC; 1 × Standard Saline Citrate is 0.15 M NaCl, 0.015 M Na citrate) then 70% ethanol, and baked under vacuum at 80°C for a few hours, or with no vacuum at 65°C overnight. At this point, the filter is ready for hybridization procedures.

The extent of hybridization is determined by a biotin assay kit commercially available from Bethesda Research Laboratory, MD. U.S.A.

As the hybridization procedure involves single-stranded DNAs, the nitrocellulose filter upon which the sample DNA is immobilised must be pretreated so that the unknown DNA and the detector probe do not bind to it indiscriminately. Such treatment commonly involves saturating available sites on the filter with protein and polysaccharide in a mixture known as Denhardt's solution (0.2% each of bovine serum albumin, ficoll and polyvinylpyrrolidone in water), in which, along with some salt and buffer (e.g., 6 × SSC, 0.1 M Tris, pH 8), a filter is soaked for a few hours at the temperature to be used for hybridization (e.g., 65°C). The presoak solution is then replaced with hybridization medium that includes denatured sample (unknown) DNA and denatured detector probe, and DNA annealing is allowed to proceed for a few hours.

Two representative hybridization conditions are: (i) 6 × SSC, 0.1 M Tris, pH 8, 65°C, the inclusion of Denhardt's solution being optional; (ii) 4 × SSC, 40% formamide, 40°C, ± Denhardt's solution. To minimize the volume of liquid needed, and to prevent evaporation, hybridizations are often done in plastic bags that have been pressed flat and sealed.

After hybridization, DNAs that have not been faithfully base paired to the selector probe are washed from the filter by a series of filter soakings in solutions that demand extensive annealing for hybrid stability to be maintained. For example, the filter is soaked in two changes of a large volume of 2 × SSC at 65°C (at which low salt concentration poorly base paired hybrids will dissociate), then in two changes of a large volume of 2 × SSC at room temperature. The filter is then dried by blotting on filter paper.

**Claims**

1. A labeled nucleic acid probe comprising (a) a nucleic acid component, (b) a nucleic acid-binding ligand photo-chemically linked to the nucleic acid component, (c) a label and (d) a spacer chemically linking (b) and (c), characterized in that the spacer is
-glycyl-glycyl-glycyl- or
$—HN—CH_2—CH_2—NH—$ or
$—NH(CH_2)_5—CO—$ or
$—NH—(CH_2)_6—NH—$ or
spermine or spermidine.

2. A probe according to claim 1, wherein the spacer is connected to at least one of (b) and (c) by a divalent radical or a coupler.

3. A probe according to claim 1, wherein the nucleic acid-binding ligand is an intercalator compound selected from acridine dyes, phenanthridines, phenazines, furoccumarins, phenothiazines and quinolines and antracyclines.

4. A probe according to claim 3, wherein the intercalator is a compound of the formula

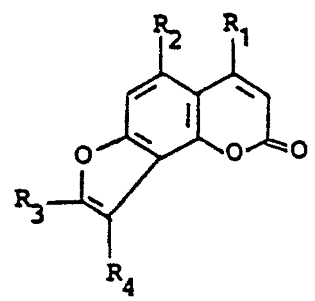

in which
$R_1$, $R_2$ and $R_3$ each independently is hydrogen or lower alkyl, and
$R_4$ is hydrogen, lower alkyl or lower alkyl substituted by hydroxy, lower alkoxy, amino, halo and/or

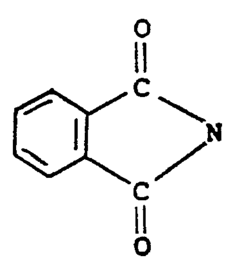

preferably 4'-aminomethyl-4,5'-dimethyl-angelicin of the formula

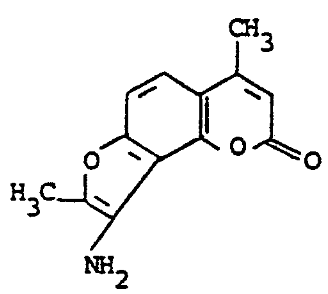

5. A probe according to claim 3, characterized in that the intercalator is a compound of the formula

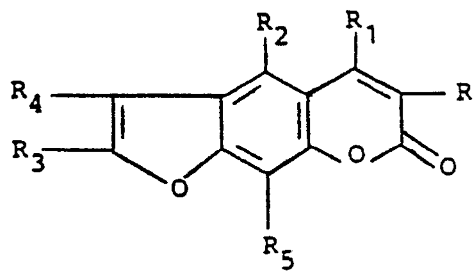

in which

R, $R_1$, $R_2$ and $R_3$ each independently is hydrogen or lower alkyl,

$R_4$ is hydrogen, lower alkyl or lower alkyl substituted by hydroxy, lower alkoxy, amino, halo and/or

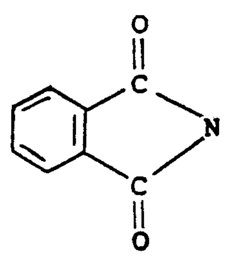

and

$R_5$ is hydrogen, hydroxy, carboxy, carbo-lower alkoxy or lower alkoxy, preferably

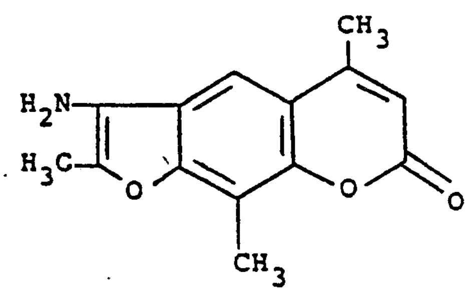

6. A method for determination of the presence of a particular nucleic acid in a test sample wherein the sample is subjected to hybridizing conditions with a labeled nucleic acid probe, and the product is assayed for the presence of said label, wherein said probe comprises (a) a nucleic acid component, (b) a nucleic acid-binding ligand photochemically linked to the nucleic acid component, (c) a label and (d) a spacer chemically linking (b) and (c), characterized in that the spacer is

-glycyl-glycyl-glycyl- or
$—HN—CH_2—CH_2—NH—$ or
$—NH(CH_2)_5—CO—$ or
$—NH—(CH_2)_6—NH—$ or
spermine or spermidine.

7. Use of a labeled nucleic acid probe in a hybridization technique said probe comprising (a) a nucleic acid component, (b) a nucleic acid-binding ligand photochemically linked to the nucleic acid component, (c) a label and (d) a spacer chemically linking (b) and (c), characterized in that the spacer is

-glycyl-glycyl-glycyl- or
$—HN—CH_2—CH_2—NH—$ or
$—NH(CH_2)_5—CO—$ or
$—NH—(CH_2)_6—NH—$ or
spermine or spermidine.

**Patentansprüche**

1. Markierte Nucleinsäure-Sonde, welche (a) eine Nucleinsäure-Komponente, (b) einen Nucleinsäure-Bindungsliganden, der fotochemisch mit der Nucleinsäure-Komponente verbunden wurde, (c) einen Marker, und (d) einen Spacer, der (b) und (c) chemisch miteinander verbindet, umfasst, dadurch gekennzeichnet, dass der Spacer

-Glycyl-Glycyl-Glycyl- oder
$—HN—CH_2—CH_2—NH—$ oder
$—NH(CH_2)_5—CO—$ oder
$—NH—(CH_2)_6—NH—$ oder
Spermin oder Spermidin
darstellt.

2. Sonde nach Anspruch 1, worin der Spacer mit mindestens einer Komponente von (b) und (c) mit Hilfe eines divalenten Restes oder einer Kupplungsverbindung verbunden ist.

3. Sonde nach Anspruch 1, worin der Nucleinsäure-Bindungsligand eine Einlagerungsverbindung, ausgewählt aus Acridin-Farbstoffen, Phenanthridinen, Phenazinen, Furocumarinen, Phenothiazinen und Chinolinen und Anthracyclinen, darstellt.

4. Sonde nach Anspruch 3, worin die Einlagerungsverbindung eine Verbindung der folgenden Formel darstellt:

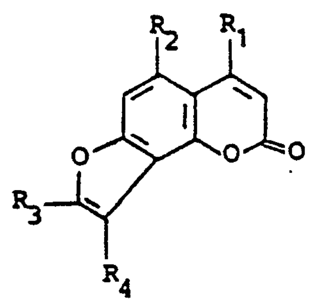

worin
$R_1$, $R_2$ und $R_3$ jeweils unabhängig voneinander Wasserstoff oder Niedrigalkyl darstellen, und
$R_4$ Wasserstoff, Niedringalkyl oder mit Hydroxy subsituiertes Niedrigalkyl, Niedrigalkoxy, Amino, Halogen und/oder

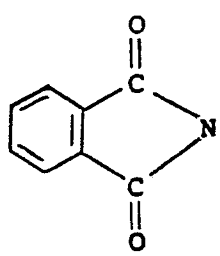

bedeutet, vorzugsweise 4'-Aminomethyl-4,5'-dimethyl-angelicin der folgenden Formel:

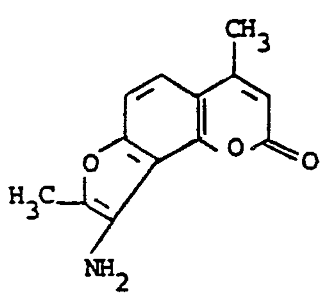

5. Sonde nach Anspruch 3, dadurch gekennzeichnet, dass die Einlagerungsverbindung eine Verbindung der folgenden Formel darstellt:

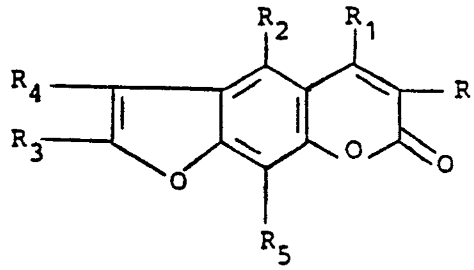

worin

R, $R_1$, $R_2$ und $R_3$ jeweils unabhängig voneinander Wasserstoff oder Niedrigalkyl bedeuten,

$R_4$ Wasserstoff, Niedringalkyl oder mit Hydroxy subsituiertes Niedrigalkyl, Niedrigalkoxy, Amino, Halogen und/oder

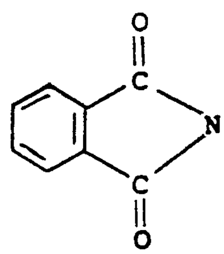

bedeutet, und

$R_5$ Wasserstoff, Hydroxy, Carboxy, Carbo-niedrigalkoxy oder Niedrigalkoxy darstellt, vorzugweise

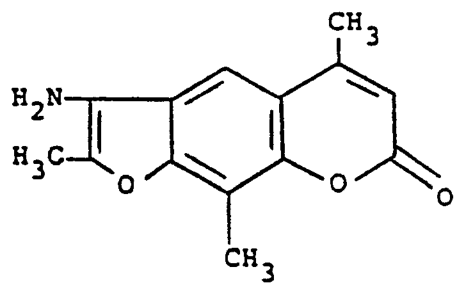

6. Verfahren zur Bestimmung der Anwesenheit einer bestimmten Nucleinsäure in einer Testprobe, wobei eine markierte Nucleinsäure-Sonde unter Hybridisierungsbedingungen auf die Probe einwirkt, und das Produkt zum Nachweis des genannten Markers einer Bestimmung unterworfen wird, und wobei die genannte Sonde (a) eine Nucleinsäure-Komponente, (b) einen Nucleinsaüre-Bindungsliganden, der fotochemisch an die Nucleinsäure-Komponente gebunden wurde, (c) einen Marker, und (d) einen Spacer, der (b) und (c) chemisch miteinander verbindet, umfasst, dadurch gekennzeichnet, dass der Spacer

-Glycyl-Glycyl-Glycyl- oder
$—HN—CH_2—CH_2—NH—$ oder
$—NH(CH_2)_5—CO—$ oder
$—NH—(CH_2)_6—NH—$ oder
Spermin oder Spermidin

darstellt.

7. Verwendung einer markierten Nucleinsäure-Sonde in einem Hybridisierungsverfahren, wobei die genannte Sonde (a) eine Nucleinsäure-Komponente, (b) einen Nucleinsäure-Bindungsliganden, der fotochemisch mit der Nucleinsäure-Komponente verbunden wurde, (c) einen Marker, und (d) einen Spacer, der (b) und (c) chemisch miteinander verbindet, umfasst, dadurch gekennzeichnet, dass der Spacer

-Glycyl-Glycyl-Glycyl- oder
$—HN—CH_2—CH_2—NH—$ oder
$—NH(CH_2)_5—CO—$ oder
$—NH—(CH_2)_6—NH—$ oder
Spermin oder Spermidin

**Revendications**

1. Produit de détection acide nucléique marqué comprenant (a) un composant acide nucléique, (b) un ligand pour liaison de l'acide nucléique lié photochimiquement au composant acide nucléique, (c) marqueur et (d) un agent d'espacement liant chimiquement (b) et (c), caractérisé en ce que l'agent d'espacement est

-glycyl-glycyl-glycyl- ou
$—HN—CH_2—CH_2—NH—$ ou
$—NH(CH_2)_5—CO—$ ou
$—NH—(CH_2)_6—NH—$ ou
spermine ou spermidine.

2. Produit de détection suivant la revendication 1, dans lequel l'agent d'espacement est relié à au moins un des (b) et (c) par un radical divalent ou un agent couplant.

3. Produit de détection suivant la revendication 1, caractérisé en ce que le ligand pour liaison de l'acide nucléique est un composé intercalant choisi parmi colorants acridines, phénanthridines, phénazines, furocoumarines, phénothiazines et quinoléines et anthracyclines.

4. Produit de détection suivant la revendication 3, dans lequel l'agent intercalant est un composé de formule

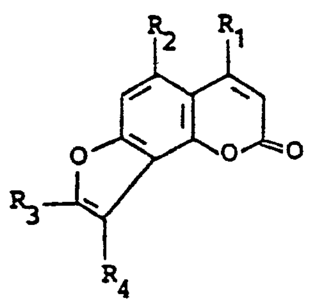

dans laquelle

$R_1$, $R_2$ et $R_3$ sont chacun indépendamment hydrogène ou alkyle inférieur, et

$R_4$ est hydrogène, alkyle, inférieur ou alkyle inférieur substitué pa hydroxy, alkoxy, inférieur, amino, halo et/ou

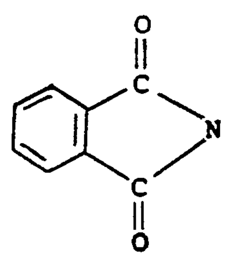

de préférence 4'-aminométhyl-4,5'-diméthyl-angélicine de formule

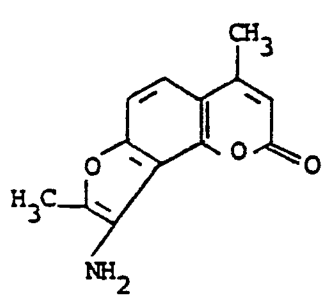

5. Produit de détection suivant la revendication 3, caractérisé en ce que l'agent intercalant est un composé

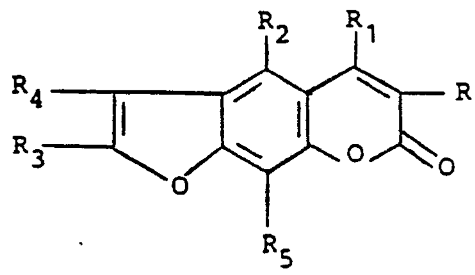

dans laquelle

R, $R_1$, $R_2$ et $R_3$ sont chacun indépendamment hydrogène ou alkyle inférieur

$R_4$ est hydrogène, alkyle, inférieur ou alkyle inférieur substitué par hydroxy, alkoxy, inférieur, amino halo et/ou

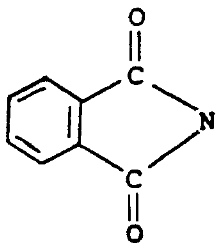

et

$R_5$ est hydrogène, hydroxy, carboxy, carbo-alkoxy inférieur ou alkoxy inférieur, de préférence

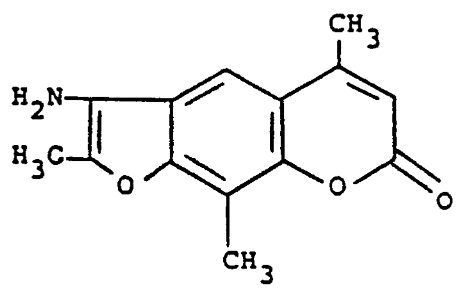

6. Procédé pour détermination de la présence d'un acide nucléique particulier dans un échantillon pour test, dans lequel l'échantillon est soumis à des conditions d'hybridation avec un produit de détection acide nucléique marqué, et le produit est analysé en ce qui concerne la présence de ce marqueur, dans lequel le produit de détection comprend (a) un composant acide nucléique, (b) un ligand pour liaison de l'acid nucléique photochimiquement lie au composant acide nucléique, (c) un marqueur et (d) un agent d'espacement liant chimiquement (b) et (c), caractérisé en ce que l'agent d'espacement est

-glycyl-glycyl-glycyl- ou
$—HN—CH_2—CH_2—NH—$ ou
$—NH(CH_2)_5—CO—$ ou
$—NH—(CH_2)_6—NH—$ ou
spermine ou spermidine.

7. Utilisation d'un produit de détection acide nucléique marqué dans un technique d'hybridation, ce produit, de détection comprenant (a) un composant acide nucléique, (b) un ligand pour liaison de l'acide nucléique lié photochimiquement au composant acide nucléique, (c) un marqueur et (d) un agent d'espacement liant chimiquement (b) et (c), caractérisé en ce que l'agent d'espacement est

-glycyl-glycyl-glycyl- ou
$—HN—CH_2—CH_2—NH—$ ou
$—NH(CH_2)_5—CO—$ ou
$—NH—(CH_2)_6—NH—$ ou
spermine ou spermidine.